# EUROPÄISCHE PATENTSCHRIFT

(11) **EP 1 196 447 B1**
(45) Veröffentlichungstag und Bekanntmachung des Hinweises auf die Patenterteilung: **18.02.2009**
(21) Anmeldenummer: 00943838.3
(22) Anmeldetag: 20.06.2000
(51) Int. Cl.: C07K 14/62, C12P 21/02, C07K 1/00

(54) **VERFAHREN ZUR STABILISIERUNG VON PROTEINEN IN KOMPLEXEN MISCHUNGEN BEI DEREN LAGERUNG IN WÄSSRIGEN LÖSUNGSMITTELN**
METHOD FOR STABILISING PROTEINS IN COMPLEX MIXTURES DURING THEIR STORAGE IN AQUEOUS SOLVENTS
PROCEDE DE STABILISATION DE PROTEINES DANS DES MELANGES COMPLEXES LORS DE LEUR STOCKAGE DANS DES SOLVANTS AQUEUX

(30) Priorität: 02.07.1999 DE 19930676
(43) Veröffentlichungstag der Anmeldung: 17.04.2002
(73) Patentinhaber: Sanofi-Aventis Deutschland GmbH, 65929 Frankfurt am Main (DE)
(72) Erfinder: RUBROEDER, Franz-Josef, D-65606 Vilmar (DE); KELLER, Reinhold, D-65812 Bad Soden (DE)
(86) Internationale Anmeldenummer: PCT/EP2000/005666
(87) Internationale Veröffentlichungsnummer: WO 2001/002435

(56) Entgegenhaltungen:
- EP-A- 0 335 554
- EP-A- 0 437 622
- EP-A- 0 906 918
- US-A- 4 371 523
- US-A- 4 937 085
- US-A- 5 756 672
- LOUGHEED W D ET AL: "Insulin aggregation in artifical delivery systems" DIABETOLOGIA,DE,BERLIN, Bd. 19, Nr. 1, Juli 1980 (1980-07), Seiten 1-9, XP002100222 ISSN: 0012-186X
- DATABASE MEDLINE [Online] US NATIONAL LIBRARY OF MEDICINE (NLM), BETHESDA, MD, US; QI H ET AL.: "STABILITY AND STABILIZATION OF INSULINOTROPIN IN A DEXTRAN FORMULATION" retrieved from STN Database accession no. AN 96148261 XP002150464 & PDA JOURNAL OF PHARMACEUTICAL SCIENCE AND TECHNOLOGY (1995 NOV-DEC) 49 (6) 289-93,

## Beschreibung

Die vorliegende Erfindung betrifft ein Verfahren zur Lagerung von Proteinen in einem wäßrigen Lösungsmittel.

Der chemische Zustand der SH-Gruppen von Proteinen oder anderer oxidationsempfindlicher Strukturen hat häufig Einfluß auf die Identität, Aktivität oder wirksame Konzentration von Proteinen. Identität bedeutet die jeweilige Faltung eines Proteins. Unter Aktivität soll eine Enzymaktivität verstanden werden. Die wirksame Konzentation eines Proteins soll der Anteil des Proteins in einer Lösung sein, der bezüglich der biologischen in vivo-Funktion korrekt gefaltet ist.

Aus der Literatur ist bekannt, daß strukturverändernde Oxidationen an Proteinen durch den Einsatz von Thiolreagenzien wie z.B. 2-Mercaptoethanol oder Cystein unterdrückt werden können.

Beispielsweise läßt sich die D-Aminosäureoxidase (DAO) durch Thiole stabilisieren. Das Flavoprotein DAO katalysiert die stereospezifische Deaminierung von D-Aminosäuren zu den entsprechenden α-Ketosäuren und Ammonium (P. Golini et al. Enzyme and Microbial Technology, 17, 1995, 324 - 329) . Allerdings kann die Zugabe von Thiolen wie z.B. Cystein die Aktivität von Proteinen auch reduzieren. Auch dieser Effekt kann mit dem Vorhandensein von Cysteinresten erklärt werden. Ein Beispiel dafür ist die Aminoacylase. Die Aminoacylase ist ein dimeres Enzym mit einem Zn²⁺- Atom pro Untereinheit. Jede Untereinheit des Enzyms enthält 2 Cystein SH-Gruppen und 2 Disulfid-Bindungen. Die chemische Modifizierung der SH-Gruppen wie das Aufbrechen der Disulfid-Bindungen kann zu einer Inaktivierung des Enzyms führen. Es konnte gezeigt werden, daß durch die Zugabe von 2-Mercaptoethanol die Aktivität der Aminoacylase reduziert wird, wohingegen nach Entfernung des 2-Mercaptoethanol durch Dialyse oder Gelfiltration die ursprüngliche Enzymaktivität fast komplett wiederhergestellt werden kann (W. Kördel u. F. Schneider, Biochem. Biophys. Acta 445, 1976, 446-457).

Für Cystein und einige Derivate konnte für bestimmte Zubereitungsformen und spezielle Anwendungen in einem gewissen Umfang eine antibakterielle, antivirale oder antifungische Aktivität nachgewiesen werden. So konnte beispielsweise gezeigt werden, daß die Zugabe von Cystein sich in gewissem Umfang als Schutz vor dem Verderb von Lebensmitteln eignet (US 4937085 A).

Mit Hilfe gentechnologischer Verfahren können rekombinante Proteine wie Insulin oder dessen Vorläufer sowie Insulinderivate, die von der abgeleiteten Gensequenz (z.B. human) abweichende Aminosäurezusammensetzungen aufweisen, in gentechnisch veränderten Mikroorganismen wie dem Bakterium Escherichia coli synthetisiert werden.

Die rekombinante Synthese in Mikroorganismen wird mit Hilfe von Expressionsvektoren durchgeführt. Diese Expressionsvektoren bestehen aus einem Vektor-Plasmid, das eine Kontrollsequenz für die Replikation des Plasmids sowie ein Selektionsgen (Antibiotikumresistenzgen, Stoffwechselmarker u.a.) enthalten sollte, in welches die codierende Region des Gens für das interessierende Protein (z.B. Insulin) unter Kontrolle eines im gewählten Mikroorganismus aktiven Promoters (für E. coli z.B. lac-Promoter) eingesetzt wurde.

Ein Verfahren zur Gewinnung rekombinanter Proteine (z.B. Insulin; Insulinderivate u.a.) unter Mitwirkung gentechnisch veränderter Mikroorganismen setzt sich aus einer Serie von Verfahrensschritten zusammen, die ineinandergreifen und aufeinander abgestimmt sein müssen.

So kann beispielsweise ein Verfahren zur Gewinnung von Humaninsulin in E. coli aus den folgenden Verfahrensschritten aufgebaut sein.
Fermentation der Mikroorganismen Zellabtrennung-Zellaufschluß-Isolierung und Zwischenlagerung des Fusionsproteins mit Cystein- Rückfaltung in die native Raumstruktur unter Ausprägung der Disulfidbrücken mit nachfolgender Abtrennung nicht wertstoffhaltiger Fremproteine -Enzymatische Spaltung zum Arginylinsulin-Basisreinigung der wässrigen Proteinlösung - 1. te chromatographische Reinigung - enzymatische Spaltung zum Humaninsulin - 2. te chromatographische Reinigung - Hochreinigung mittels HPLC - Umkristallisation - Trocknung.

Die Vielzahl der durchgeführten Einzeimaßnahmen führt in aller Regel zu erheblichen Verlusten bei der Gesamtausbeute, da bei jedem Schritt Einbußen, bedingt durch die spezifische Ausbeute des gewählten Verfahrensschritts, unumgänglich sind.

Durch Optimierung von Zwischenschritten kann die Gesamtausbeute verbessert werden. An solchen Verfahren besteht ein beträchtliches Interesse, damit eine bessere wirtschaftliche Verwertung der eingesetzten Ressourcen sowie eine Verringerung der Umweltbelastungen ermöglicht wird.

Beispielsweise wird in EP 0906918 ein verbessertes Verfahren zur Gewinnung eines Vorläufers von Insulin oder Insulinderivaten mit korrekt verbundenen Cystinbrücken in Gegenwart von Cystein oder Cysteinhydrochlorid und von einem chaotropen Hilfsstoff beschrieben.

Insulinderivate sind Derivate von natürlich vorkommenden Insulinen, nämlich Humaninsulin oder tierischen Insulinen. Diese Insulinderivate unterscheiden sich von dem ansonsten gleichen natürlich vorkommenden Insulin durch das Fehlen und/oder den Austausch wenigstens eines in dem betreffenden natürlich vorkommenden Insulin auftretenden Aminosäurerestes durch einen anderen genetisch kodierbaren Aminosäurerest und/oder die Addition von wenigstens einem genetisch kodierbaren Aminosäurerest.

Bei der Lagerung von Proteinen kommt es gewöhnlich zur Abnahme der wirksamen Konzentrationen.

Die Lagerung von Produktionsergebnissen zwischen einzelnen Verfahrensschritten kann aus verschiedenen Gründen notwendig sein. So kann es beispielsweise vorkommen, daß die Verarbeitungskapazität eines nachgeschalteten technischen Weiterverarbeitungsschritts nicht ausreicht, um die gesamte Produktmenge des vorhergehenden Verfahrensschritts aufzunehmen.

Der Zeitbedarf für eine Zwischenlagerung kann unterschiedlich lange sein. Bedingt durch Kapazität, Abstimmungsbedarf technischer Aggregate, benötigte Nachlieferung von Chemikalien oder Vorrichtungen oder aus anderen Gründen kann sich die erforderliche Zwischenlagerung auf mehrere Wochen ausdehnen.

Ein effektiver Weg zur Ausbeutesteigerung eröffnet sich, wenn es gelingt, die bei der unumgänglichen Zwischenlagerung von Produkten einzelner Verfahrensschritte vor deren Weiterverarbeitung auftretenden Verluste an wirksamem Protein in möglichst engen Grenzen zu halten.

Bisher nicht beschrieben ist die Verwendung von Cystein oder Derivaten zur Eindämmung von Ausbeuteverlusten an wirksamem Protein bei der Zwischenlagerung von Produktionsergebnissen unterschiedlicher Komposition und Zustandsfassung aus Verfahrenseinzelschritten industrieller Produktionsverfahren unter Einschluß biologischer Komponenten, beispielsweise unter Beteiligung von Enzymkatalysatoren oder gentechnisch veränderter Mikroorganismen. Solche Verfahren werden beispielsweise bei der Herstellung von Insulin angewendet.

Die zwischenzulagernden Produktionsergebnisse können je nach Verfahrensstufe aus unterschiedlich vielen, komplex zusammengesetzten Makromolekülen biologischer Natur (Proteine, DNA, Fette), Mikroorganismen, Puffersubstanzen und Ausgangsprodukten und anderem bestehen.

Das Produktionsverfahren zielt in aller Regel auf möglichst einheitliche Darsteliung eines Stoffes, im Falle der Insulinproduktion auf Insulin. Wenn Produktionsergebnisse zwischengelagert werden müssen, kommt es im Falle der Herstellung eines Proteins, wie z.B. Insulin, regelmäßig zum Verlust an wirksamer Konzentration dieses Proteins.

Unter Lagerung eines Proteins soll jede Aufbewahrung des Proteins verstanden werden, ungeachtet von der Volumenmenge, in der das Protein vorliegt, der Zeitdauer, über die die Aufbewahrung vorgenommen wird, oder den Temperaturbedingungen, unter denen sie erfolgt. Die Lagerung von Proteinen erfolgt normalerweise in wäßrigen Lösungen.

Eine wäßrige Lösung eines Proteins kann neben Bestandteilen von Nährmedien zum Anzüchten von Mikroorganismen in definierter Form oder als Vollmedien mit insbesondere Kohlenstoffquellen oder Stickstoffquellen, Aminosäuren und anorganischen Salzen und Spurenelementen, auch Pufferanteile unterschiedlicher chemischer Puffertypen, sowie Makromoleküle biologischer Herkunft wie DNA oder Fette, desweiteren organische oder anorganische Verbindungen wie z.B. Na-dodecylsulfat oder K-acetat und auch Anteile von Lösungsmittel unterschiedlicher Priorität wie Methanol oder Petrolether enthalten.

Lougheed, W. D. et al. (1980) Diabetologia, 19, 1-9, No. 1 beschreibt die Insulinaggregation in einem artifiziellen Zuführungssystem sowie ein Verfahren, bei dem Insulin für 30 Tage in Anwesenheit von Cystein gelagert wurde.

Die vorliegende Erfindung betrifft nun ein Verfahren zur Lagerung eines Proteins, bei der die zeitliche Abnahme der wirksamen Konzentration des Proteins verzögert wird, in einem wässrigen Lösungsmittel, wobei Cystein zu dem wässrigen Lösungsmittel zugegeben wird und die Konzentration von Cystein in der wässrigen Proteinlösung im Bereich von 100 bis 500 mM liegt.

Das Verfahren eignet sich zur Stabilisierung von Verlangsamung der zeitlichen Abnahme der wirksamen Konzentration eines Proteins während dessen Lagerung in wäßrigen Lösungen. Das Verfahren kann angewendet werden beispielsweise bei der Produktion von Proteinen durch Mikroorganismen. Als Mikroorganismen bieten sich unter anderem vor allem Bakterien, hierbei insbesondere Escherichia coli, Hefen, wie vor allem Saccharomyces cerevisiae oder Pichia pastoris, sowie Insektenzellen an. Diese Mikroorganismen können in der bevorzugten Ausführungsform mit Expressionsvektorkonstrukten zur induzierten oder konstitutiven Expression besagten Proteins transformiert sein. Die Mikroorganismen werden angezüchtet und nach Synthese des Proteins aufgeschlossen. Als Aufschlußmethode kommen prinzipiell alle Verfahren in Frage, die zur Freisetzung des Proteins aus den Bakterien geeignet sind. Anwendbar als Aufschlußverfahren sind z.B. Ultraschall, chemische Methoden unter Verwendung von K-acetat, Na-dodecylsulfat oder Lysozym, Erhitzen der Mikroorganismen oder die French press. Der Aufschluß der Mikroorganismen kann unterbleiben, wenn das herzustellende Protein direkt in das Medium ausgeschieden wird. Prinzipiell ist das Verfahren auch auf ausgeschleuste Proteine anwendbar.

Die komplexen Proteinmischungen, die entstehen, wenn die Mikroorganismen aufgeschlossen werden oder Protein direkt in das Medium ausschleusen, liegen gewöhnlich in wäßriger Lösung vor. Als Proteine werden Insulin, ein Insulinderivat und/oder ein Vorläufer desselben verwendet. In der wässrigen Lösung können die Proteine gelöst sein oder in suspendierter Form vorhanden sein. Die Aufbewahrung dieser Proteinlösungen erfolgt vorzugsweise zwischen 0°C - 50°C oder zwischen 5°C - 30°C oder bei 5°C. Zur Verzögerung der Inaktivierung wird Cystein in diese Proteinmischung gegeben. Die Konzentration von Cystein in der Proteinmischung beträgt 150 - 220 mM. Bevorzugt wird 170mM zugegeben. Proteine in komplexen Moleküllösungen können auf diese Weise bis zu mehreren Wochen mit nur geringer Abnahme der wirksamen Proteinkonzentration gelagert werden. Das Verfahren ist anwendbar für die Synthese heterologer Proteine, insbesondere in Mikroorganismen, nach deren Aufschluß mit anschließender Reinigung und eventueller Renaturierung bevorzugt zur Herstellung von Insulin und dessen Vorläufer.

Als Proteine zur Lagerung in einem Verfahren der Erfindung werden vorzugsweise Insulin oder Insulinderivate verwendet.

Die vorliegende Erfindung betrifft ferner ein Verfahren zur Herstellung eines heterologen Proteins, umfassend die Expression des heterologen Proteins oder dessen Vorstufe in einem transformierten Mikroorganismus, welches heterologe Protein dann mittels eines Verfahrens gemäß der vorliegenden Erfindung gelagert wird. Dieses heterologe Protein oder eine Vorstufe desselben wird gegebenenfalls anschließend renaturiert, von Leadersequenzen bereinigt oder in sonstiger Weise prozessiert und schließlich nach Reinigung und Isolierung zum gewünschten Produkt dargestellt. Als heterologe Proteine kommen bevorzugt Insulin oder Insulinderivate sowie Vorläufer von Insulin infrage.

### Beispiele:

Während der Implementierung der Verfahren zur Herstellung von Insulin wurde gefunden, daß durch die Zugabe von Cystein zum Fusionsprotein das Produkt über Monate lagerstabil bleibt. Dagegen verliert das nicht vorbehandelte Produkt bereits nach wenigen Tagen irreversibel einen Teil seiner Aktivität.

In den folgenden Beispielen werden die Vorläufermoleküle des humanen Insulins sowie eines Insulinderivats verwendet. Struktur und Aminosäuresequenz sind in EP 906 918 mit Sequenzprotokollen offenbart. Der Vorläufer des humanen Insulins hat die Sequenz von natürlich vorkommendem humanem Insulin. Der Vorläufer des Insulinsderivats enthält an Position 21 der A-Kette ein Glyzin an Stelle eines Arginis und am C-terminalen Ende der B-Kette an den Positionen 31 und 32 zwei Argininmoleküle.

Fusionsproteine der Insuline können durch Fermentation von genetisch modifizierten E. coli-Zellen gemäß EP 0 489 780 und EP 0 906 918 hergestellt werden. Man gewinnt dabei eine Proteinsuspension, die etwa 20 bis 25 % Trockenmasse und 40 bis 50 % an faltbarem Insulin enthält.

Für die bezweckte Stabilisierung des Proteins mittels Cystein werden zu ca. 2500 kg dieser Proteinsuspension (entsprechend einer Fermentationscharge) 75 kg Cysteinhydrochlorid*H₂O innerhalb von 20 Minuten unter starkem Rühren eingetragen. Der pH-Wert fällt dabei von 7,0 auf 2,5. Die Suspension wird im pH-Bereich um 5 sehr pastös, um ab pH 4 wieder gut rührbar zu werden. Unter diesen Versuchsbedingungen stellt sich eine Cysteinhydrochloridkonzentration von 170 mM in der Proteinsuspension ein. Die Proteinsuspension wird 60 Min. nachgerührt, danach kann sie ohne weiteres Rühren bis zur Aufarbeitung stehen bleiben.

### Beispiel 1:

Zum experimentellen Beleg der konservierenden Wirkung von Cystein wurden folgende Laborversuche durchgeführt:

Fusionsprotein des humanen Insulins und Fusionsprotein des Insulinderivats hergestellt gemäß EP 906 918 wurden mit und ohne Cystein bei 5°C und bei Raumtemperatur bis zu 2 Monaten gelagert. Den Ansätzen wurden während des Versuchs Aliquots entnommen. Das Fusionsprotein wurde durch reduktive Faltung in die präpro-Form des humanen Insulins bzw. präpro-Form des Insulinderivats überführt (EP 906 918). Ansätzen, die ohne Cysteinhydrochlorid-monohydrat gelagert wurden, wurde die zur Faltung notwendige Menge Cystein ca. 1 Stunde vor Beginn der Faltung zugefügt. Bei den anderen Ansätzen, die das Cystein in Form des Konservierungsmittels enthielten, war das nicht mehr erforderlich.

Die Umsetzung zur präpro-Form des humanen Insulins bzw. präpro-Form des Insulinderivats wurde mittels HPLC bestimmt.

### HPLC-Analyse:

0,5 g Protein werden in 40 ml einer Lösung aus 6 M Guanidinhydrochlorid, 50 mM Tris, pH 8,5 mM Ethylendiamintetraacetat (EDTA), 1 % 2-Mercaptoethanol, 10 mM Dithiothreitol bei 95°C für 2 Min. gelöst und dann bei 14 000 g für 20 Min. zentrifugiert. Von dem klaren Überstand werden 0,02 ml auf eine Hochdruckflüssigchromatographiesäule aufgetragen.
Säule: ®Nucleogel RP 300 - 5/46 (Macherey & Nagel, Aachen, Deutschland)
Gradient: Puffer A: 0,1 % Trifluoressigsäure (TFA)
   Puffer B: 0,09 % TFA in Acetonitril
Temperatur: 55°C
Gesamtlaufzeit: 40 Min.

Das Gradient ist gekennzeichnet durch die folgende Menge von Puffer B nach den entsprechenden Laufzeiten: 10 Min. 25 %, 12 Min. 60 %,13 Min. 90 %,
15 Min. 100 %.
Fluß: 1 ml/Min.
Detektion: 215 nm

### Ergebnis der Versuche

**Tabelle 1: Lagern von Fusionsprotein des humanen Insulins bei 5°C, Wertstoff (mg/L) nach der Faltung (Wertstoff bedeutet gefaltetes humanes Insulin)**

| | 1. Tag | 1 Woche | 2 Wochen | 4 Wochen | 8 Wochen |
|---|---|---|---|---|---|
| Mit Cystein | 850 | 831 | 867 | 845 | 825 |
| Ohne Cystein | 879 | 827 | 790 | 712 | 625 |

**Tabelle 2: Lagern von Fusionsprotein des humanen Insulins bei Raumtemperatur, Wertstoff (mg/L) nach der Faltung**

| | 1. Tag | 1 Woche | 2 Wochen | 4 Wochen | 8 Wochen |
|---|---|---|---|---|---|
| Mit Cystein | 792 | 817 | 800 | 785 | 790 |
| Ohne Cystein | 812 | 654 | 312 | %* | %* |

| | | | | | |
|---|---|---|---|---|---|
| *Ansetzen wegen starker Fäulnisprozesse nicht möglich | | | | | |

**Tabelle 3: Lagern von Fusionsprotein des Insulinderivats bei 5°C, Wertstoff (mg/L) nach der Faltung (Werkstoff bedeutet gefaltetes Insulinderivat)**

| | 1. Tag | 1 Woche | 2 Wochen | 4 Wochen | 8 Wochen |
|---|---|---|---|---|---|
| Mit Cystein | 590 | 553 | 540 | 573 | 552 |
| Ohne Cystein | 612 | 580 | 549 | 518 | 404 |

**Tabelle 4: Lagern von Fusionsprotein des Insulinderivats bei Raumtemperatur, Wertstoff (mg/L) nach der Faltung**

| | 1. Tag | 1 Woche | 2 Wochen | 4 Wochen | 8 Wochen |
|---|---|---|---|---|---|
| Mit Cystein | 643 | 667 | 680 | 649 | 653 |
| Ohne Cystein | 597 | 547 | 420 | 271 | %* |

| | | | | | |
|---|---|---|---|---|---|
| *Ansetzen wegen starker Fäulnisprozesse nicht möglich | | | | | |

Durch Zugabe von Cystein oder Cysteinhydrochlorid werden Lagerzeiten bis zu 2 Monaten ohne signifikanten Aktivitätsverlust ermöglicht.

## Patentansprüche

1. Verfahren zur Lagerung eines Proteins, bei der die zeitliche Abnahme der wirksamen Konzentration des Proteins verzögert wird, in einem wässrigen Lösungsmittel, **dadurch gekennzeichnet, daß** Cystein zu dem wässrigen Lösungsmittel zugegeben wird, wobei die Konzentration von Cystein in der wässrigen Proteinlösung im Bereich von 150 bis 220 mM liegt und **dadurch gekennzeichnet, dass** als Protein Insulin, ein Insulinderivat und/oder ein Vorläufer desselben verwendet wird.

2. Verfahren nach Anspruch 1, **dadurch gekennzeichnet, dass** das Protein ein in einem Mikroorganismus hergestelltes heterologes Protein ist.

3. Verfahren nach Anspruch 2, **dadurch gekennzeichnet, dass** als Mikroorganismus ein Bakterium verwendet wird.

4. Verfahren nach Anspruch 3, **dadurch gekennzeichnet, dass** als Bakterium Escherichia coli verwendet wird.

5. Verfahren nach Anspruch 2, **dadurch gekennzeichnet, dass** als Mikroorganismus eine Hefe verwendet wird.

6. Verfahren nach Anspruch 5, **dadurch gekennzeichnet, dass** als Hefe Saccharomyces cerevisiae verwendet wird.

7. Verfahren nach Anspruch 5, **dadurch gekennzeichnet, dass** als Hefe Pichia pastoris verwendet wird.

8. Verfahren nach Anspruch 2, **dadurch gekennzeichnet, dass** als Mikroorganismus Insektenzellen verwendet werden.

9. Verfahren nach einem oder mehreren der Ansprüche 2 bis 8, **dadurch gekennzeichnet, dass** der Mikroorganismus das Protein mittels eine Expressionsvektorkonstrukts herstellt.

10. Verfahren nach einem oder mehreren der Ansprüche 1 bis 9, **dadurch gekennzeichnet, dass** das Protein in gelöster Form vorliegt.

11. Verfahren nach einem oder mehreren der Ansprüche 1 bis 9, **dadurch gekennzeichnet, dass** das Protein in Suspension vorliegt.

12. Verfahren nach einem oder mehreren der Ansprüche 1 bis 11, **dadurch gekennzeichnet, dass** die Konzentration von Cystein in der wässrigen Proteinlösung 170 mM beträgt.

13. Verfahren nach einem oder mehreren der Ansprüche 1 bis 12, **dadurch gekennzeichnet, dass** die Lagerung des Proteins bei 0°C bis 50°C erfolgt.

14. Verfahren nach Anspruch 13, **dadurch gekennzeichnet, dass** die Lagerung des Proteins bei 5°C bis 30°C erfolgt.

15. Verfahren nach Anspruch 14, **dadurch gekennzeichnet, dass** die Lagerung des Proteins bei 5°C erfolgt.

16. Verfahren zur Herstellung eines heterologen Proteins, umfassend die Expression des heterologen Proteins oder dessen Vorstufe in einem transformierten Mikroorganismus, gegebenenfalls den Aufschluss des Mikroorganismus oder Isolierung des heterologen Proteins oder dessen Vorstufe aus dem Kulturmedium, nachfolgende Lagerung mittels eines Verfahrens gemäß einem oder mehreren der Ansprüche 1 bis 15 und gegebenenfalls die nachfolgende Renaturierung des heterologen Proteins oder dessen Vorstufe, gegebenenfalls Abspaltung von Leadersequenzen und von nur in der Vorstufe des heterologen Proteins auftretenden sonstigen Sequenzen und schließlich die Reinigung und Isolierung des heterologen Proteins.

17. Verfahren nach Anspruch 16, **dadurch gekennzeichnet, dass** das heterologe Protein ein tierisches Insulin oder Humaninsulin ist.

## Claims

1. A process for the storage of a protein, in which the temporal decrease in the effective concentration of the protein is delayed, in an aqueous solvent, **characterized in that** cysteine is added to be aqueous solvent, the concentration of cysteine in the aqueous protein solution being in the range from 150 to 220 mM and **characterized in that** the protein used is insulin, an insulin derivative and/or a precursor thereof.

2. The process as claimed in claim 1, **characterized in that** the protein is a heterologous protein prepared in a microorganism.

3. The process as claimed in claim 2, **characterized in that** the microorganism used is a bacterium.

4. The process as claimed in claim 3, **characterized in that** the bacterium used is Escherichia coli.

5. The process as claimed in claim 2, **characterized in that** the microorganism used is a yeast.

6. The process as claimed in claim 5, **characterized in that** the yeast used is Saccharomyces cerevisiae.

7. The process as claimed in claim 5, **characterized in that** the yeast used is Pichia pastoris.

8. The process as claimed in claim 2, **characterized in that** the microorganisms used are insect cells.

9. The process as claimed in one or more of claims 2 to 8, **characterized in that** the microorganism prepares the protein by means of an expression vector construct.

10. The process as claimed in one or more of claims 1 to 9, **characterized in that** the protein is present in dissolved form.

11. The process as claimed in one or more of claims 1 to 9, **characterized in that** the protein is present in suspension.

12. The process as claimed in one or more of claims 1 to 11, **characterized in that** the concentration of cysteine in the aqueous protein solution is 170 mM.

13. The process as claimed in one or more of claims 1 to 12, **characterized in that** the storage of the protein takes place at 0°C to 50°C.

14. The process as claimed in claim 13, **characterized in that** the storage of the protein takes place at 5°C to 30°C.

15. The process as claimed in claim 14, **characterized in that** the storage of the protein takes place at 5°C.

16. A process for the preparation of a heterologous protein, comprising the expression of the heterologous protein or its precursor in a transformed microorganism, if appropriate the disruption of the microorganism or isolation of the heterologous protein or its precursor from the culture medium, subsequent storage by means of a process as claimed in one or more of claims 1 to 15 and, if appropriate, the subsequent renaturation of the heterologous protein or its precursor, if appropriate removal of leader sequences and of other sequences only occurring in the precursor of the heterologous protein and finally the purification and isolation of the heterologous protein.

17. The process as claimed in claim 16, **characterized in that** the heterologous protein is an animal insulin or human insulin.

## Revendications

1. Procédé pour le stockage d'une protéine, par lequel est retardée la diminution avec le temps de la concentration efficace de la protéine, dans un solvant aqueux, **caractérisé en ce qu'**on ajoute de la cystéine au solvant aqueux, la concentration de la cystéine dans la solution aqueuse de protéine étant dans la plage de 150 à 220 mM et **caractérisé en ce qu'**on utilise comme protéine l'insuline, un dérivé d'insuline et/ou un précurseur de celle-ci.

2. Procédé selon la revendication 1, **caractérisé en ce que** la protéine est une protéine hétérologue produite dans un micro-organisme.

3. Procédé selon la revendication 2, **caractérisé en ce qu'**on utilise en tant que micro-organisme une bactérie.

4. Procédé selon la revendication 3, **caractérisé en ce qu'**on utilise en tant que bactérie *Escherichia coli.*

5. Procédé selon la revendication 2, **caractérisé en ce qu'**on utilise en tant que micro-organisme une levure.

6. Procédé selon la revendication 5, **caractérisé en ce qu'**on utilise comme levure *Saccharomyces cerevisiae.*

7. Procédé selon la revendication 5, **caractérisé en ce qu'**on utilise comme levure *Pichia pastoris.*

8. Procédé selon la revendication 2, **caractérisé en ce qu'**on utilise en tant que micro-organisme des cellules d'insecte.

9. Procédé selon une ou plusieurs des revendications 2 à 8, **caractérisé en ce que** le micro-organisme produit la protéine au moyen d'une construction de vecteur d'expression.

10. Procédé selon une ou plusieurs des revendications 1 à 9, **caractérisé en ce que** la protéine se trouve sous forme dissoute.

11. Procédé selon une ou plusieurs des revendications 1 à 9, **caractérisé en ce que** la protéine est présente en suspension.

12. Procédé selon une ou plusieurs des revendications 1 à 11, **caractérisé en ce que** la concentration de cystéine dans la solution aqueuse de protéine est de 170 mM.

13. Procédé selon une ou plusieurs des revendications 1 à 12, **caractérisé en ce que** le stockage de la protéine s'effectue à une température de 0 °C à 50°C.

14. Procédé selon la revendication 13, **caractérisé en ce que** le stockage de la protéine s'effectue à une température de 5 °C à 30 °C.

15. Procédé selon la revendication 14, **caractérisé en ce que** le stockage de la protéine s'effectue à 5 °C.

16. Procédé pour la production d'une protéine hétérologue, comprenant l'expression de la protéine hétérologue ou de son précurseur dans un micro-organisme transformé, éventuellement la désintégration du micro-organisme ou l'isolement de la protéine hétérologue ou de son précurseur à partir du milieu de culture, le stockage subséquent au moyen d'un procédé selon une ou plusieurs des revendications 1 à 15 et éventuellement la renaturation subséquente de la protéine hétérologue ou de son précurseur, éventuellement la séparation de séquences de tête et d'autres séquences n'apparaissant que dans le précurseur de la protéine hétérologue et enfin la purification et l'isolement de la protéine hétérologue.

17. Procédé selon la revendication 16, **caractérisé en ce que** la protéine hétérologue est une insuline animale ou l'insuline humaine.
